(19) Europäisches Patentamt
European Patent Office
Office européen des brevets

(11) Veröffentlichungsnummer : **0 256 331 B1**

(12) # EUROPÄISCHE PATENTSCHRIFT

(45) Veröffentlichungstag der Patentschrift : **06.11.91 Patentblatt 91/45**

(51) Int. Cl.$^5$ : **C07D 249/18,** C07D 249/20, C09B 62/507

(21) Anmeldenummer : **87110514.4**

(22) Anmeldetag : **21.07.87**

(54) **N1-Substituierte 1H-Benzo-triazol-hydroxyethyl-sulfon-Verbindungen, Verfahren zu ihrer Herstellung und ihre Verwendung zur Herstellung von Farbstoffen.**

(30) Priorität : **26.07.86 DE 3625386**

(43) Veröffentlichungstag der Anmeldung : **24.02.88 Patentblatt 88/08**

(45) Bekanntmachung des Hinweises auf die Patenterteilung : **06.11.91 Patentblatt 91/45**

(84) Benannte Vertragsstaaten : **BE CH DE ES FR GB IT LI**

(56) Entgegenhaltungen :
**EP-A- 0 141 996**
**EP-A- 0 222 098**

(73) Patentinhaber : **HOECHST AKTIENGESELLSCHAFT**
**Postfach 80 03 20**
**W-6230 Frankfurt am Main 80 (DE)**

(72) Erfinder : **Schwaiger, Günther, Dr.**
**Johannesallee 41**
**W-6230 Frankfurt am Main (DE)**
Erfinder : **Springer, Hartmut, Dr.**
**Am Erdbeerstein 27**
**W-6240 Königstein/Taunus (DE)**

**EP 0 256 331 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung liegt auf dem technischen Gebiet der Zwischenprodukte.

Die vorliegende Erfindung betrifft Verbindungen der allgemeinen Formel (1)

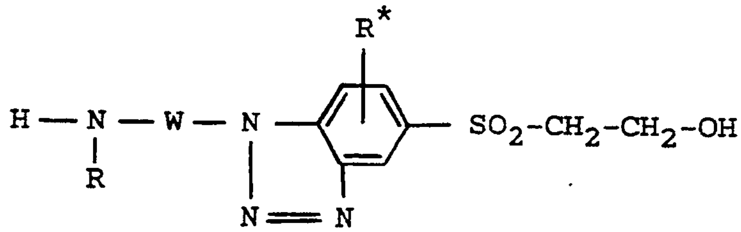

(1)

in welcher

R ein Wasserstoffatom oder eine Alkylgruppe von 1 bis 6 C-Atomen ist, die durch einen oder zwei Substituenten, bevorzugt einen Substituenten, aus der Gruppe Hydroxy, Sulfo, Carboxy, Phosphono und Cyano substituiert sein kann, bevorzugt ein Wasserstoffatom ist,

W den 1,2-Ethylen-, 1,3-Propylen-, 1,4-Butylen-, 1,5-Pentylen-, 1,6-Hexylen-, 1,2-Propylen-, 1,2-Butylen-, 1,3-Butylen-, 2-Methyl-1,3-propylen-, 2-Hydroxy-1,3-propylen-, 2-Carboxy-1,5-pentylen- oder den 2-Phenyl-1,3-propylen-Rest bedeutet oder ein im Benzolrest sulfosubstituierter 2-Phenyl-1,3-propylen-Rest ist oder einen Rest der allgemeinen Formel (1a), (1b), (1c) oder (1d)

$$-(CH_2)_2-X-(CH_2)_2- \qquad (1a)$$

$$-(CH_2)_3-X-(CH_2)_3- \qquad (1b)$$

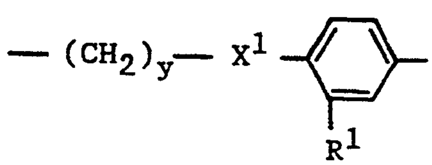

(1c)

$$-(CH_2-CH_2-NH)_n-CH_2-CH_2- \qquad (1d)$$

ist, in welchen

X ein Sauerstoffatom, ein Schwefelatom, eine Sulfonylgruppe oder eine Gruppe der Formel -NH- , $-N(CH_3)-$ oder $-N(COCH_3)-$ bedeutet,

$R^1$ für ein Wasserstoffatom oder eine Sulfogruppe steht,

n die Zahl 2, 3 oder 4 ist,

$X^1$ für die Gruppe -NH- oder für ein Sauerstoffatom steht und

y die Zahl 2 oder 3 bedeutet,

oder der 1,3-Cyclohexylen-, 1,4-Cyclohexylen-, Bis-(1,4-cyclohexylen)-methan-, 1,8-Di-methylen-naphthalin-, 1,4-Di-methylen-benzol-, 1,3-Di-methylen-benzol-, 1,4-Bis-(1',2'-ethylen)-piperidin-, 1,4-Phenylen- oder 1,3-Phenylen-Rest oder ein Rest der allgemeinen Formel (1e) oder (1f)

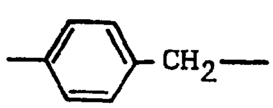

(1e)

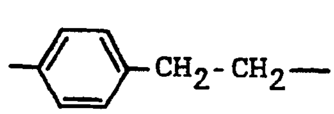

(1f)

ist, bevorzugt hiervon der 1,2-Ethylen-, 1,3-Propylen-, 1,4-Butylen-, Isopropylen-, para-phenylen- und metaphenylenrest ist, und

R* ein Wasserstoffatom oder eine Alkylgruppe von 1 bis 6 C-Atomen, bevorzugt von 1 bis 4 C-Atomen, wie insbesondere hiervon Methyl und Ethyl, eine Alkoxygruppe von 1 bis 5 C-Atomen, bevorzugt von 1 bis 4 C-Atomen, wie insbesondere hiervon Methoxy oder Ethoxy, ein Halogenatom, wie Fluor und Brom und insbesondere Chlor, oder eine Carboxy- oder Sulfogruppe bedeutet,

sowie deren Verwendung zur Herstellung von faserreaktiven Farbstoffen.

Im vorstehenden wie nachstehenden bedeutet eine Carboxygruppe eine Gruppe der allgemeinen Formel -COOM , eine Sulfogruppe eine Gruppe der allgemeinen Formel $-SO_3M$ und eine Phosphonogruppe eine Gruppe der allgemeinen Formel $-PO_3M_2$ ,
in welchen
M ein Wasserstoffatom oder ein Alkalimetall, wie Natrium, Kalium oder Lithium, bedeutet.

Die vorliegende Erfindung betrifft weiterhin ein Verfahren zur Herstellung der Verbindungen der allgemeinen Formel (1), das dadurch gekennzeichnet ist, daß man in einer Verbindung der allgemeinen Formel (2)

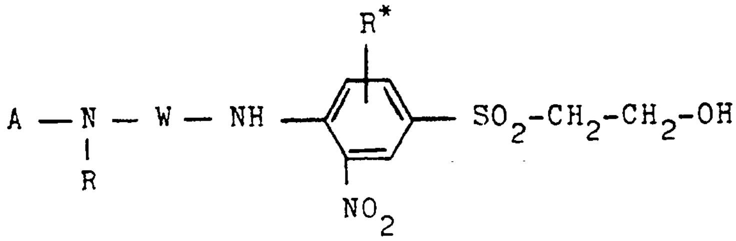 (2)

in welcher R, R* und W die obengenannten Bedeutungen haben und A ein Wasserstoffatom oder ein Acylrest, wie ein Alkanoylrest von 2 bis 5 C-Atomen, wie der Acetylrest, oder ein Benzoylrest, ist, wobei A im Falle, daß W einen aromatisch-carbocyclischen Rest bedeutet, zwingend einen Acylrest darstellt, deren Nitrogruppe in an und für sich üblicher und zu bekannten Verfahren analoger Weise zur Aminogruppe reduziert und die hieraus erhaltene Verbindung der allgemeinen Formel (3)

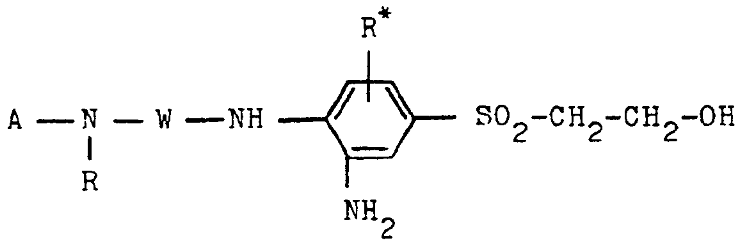 (3)

in welcher A, R, R* und W die obengenannten Bedeutungen haben, in an und für sich üblicher Verfahrensweise der Diazotierung diazotiert, so bspw. mittels Natriumnitrit in wäßrig-saurem, vorzugsweise salzsaurem, Medium bei einer Temperatur zwischen -10°C und +20°C, und gegebenenfalls im Falle, daß in Formel (3) die Gruppe A einen Acylrest bedeutete, die als Acylderivat erhaltene Benzotriazolverbindung der allgemeinen Formel (1) anschließend zur Benzotriazolverbindung der allgemeinen Formel (1) analog bekannten Methoden entacyliert, so bspw. in wäßrigem Medium bei einer Temperatur zwischen etwa 90 und 100°C und bei einem pH von größer als 12, wie mittels einer die drei- bis sechsfache Menge an NaOH enthaltenden Natronlauge, bevorzugt jedoch in saurem, bspw. salzsaurem, wäßrigen Medium bei einem pH-Wert von Kleiner als 2. Mit der Diazotierung der Verbindungen der Formel (3) erfolgt Ringschluß der sich bildenden Diazoniumgruppe mit der ortho-ständigen Aminogruppe.

Die Verbindungen der allgemeinen Formel (2) lassen sich ebenfalls in erfindungsgemäßer Weise herstellen, indem man eine Verbindung der allgemeinen Formel (4)

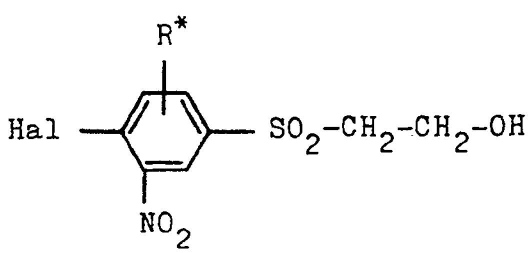 (4)

in welcher R* die obengenannte Bedeutung besitzt und Hal ein Fluor- oder Bromatom oder bevorzugt ein Chloratom bedeutet, mit einer Verbindung der allgemeinen Formel (5)

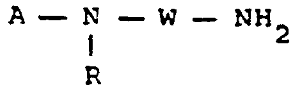

(5)

in welcher A, R und W die obengenannten Bedeutungen haben, in einem für diese Reaktanten geeigneten Lösemittel in Gegenwart eines säurebindenden Mittels bei einer Temperatur zwischen 30 und 120°C, vorzugsweise zwischen 70 und 90°C, umsetzt.

Die Ausgangsverbindungen der allgemeinen Formel (4) sind an und für sich bekannt (s. beispielsweise Deutsche Patentschrift 859 462, Beispiel 5); noch nicht per se beschriebene Verbindungen entsprechend der allgemeinen Formel (4) lassen sich analog zu den bekannten Verbindungen in der dem Fachmann geläufigen Weise herstellen, so durch Nitrierung einer entsprechenden (β-Hydroxyethylsulfonyl)-chlorbenzol-Verbindung oder durch Reduktion einer entsprechenden 4-Chlor-3-nitrobenzolsulfochlorid-Verbindung mittels Natriumsulfit zur entsprechenden Sulfinsäure und anschließender Oxethylierung der Sulfinsäure zur β-HydroxyethylsulfonylVerbindung synthetisieren.

Ausgangsverbindungen entsprechend der allgemeinen Formel (5) sind beispielsweise 1,2-Diamino-ethan, 1,3-Diamino-propan, 1,4-Diamino-butan, 1,5-Diamino-pentan, 1,6-Diamino-hexan, 1,2-Diamino-propan, 1,2-Diamino-butan, 1,3-Diamino-butan, die N-Acyl-, wie die N-Acetyl- und N-Benzoyl-Verbindungen, des 1-Amino-3-methylamino-propans, das 1,3-Diamino-2-methyl-propan, das 1,3-Diamino-2-hydroxy-propan, das 1,5-Diamino-2-carboxy-pentan, das 1,3-Diamino-2-phenyl-propan oder dessen im Benzolrest sulfosubstituiertes Derivat, des weiteren Verbindungen entsprechend einer allgemeinen Formel (a), (b), (c) und (d)

$H_2N\text{-}(CH_2)_2\text{-}X\text{-}(CH_2)_2\text{-}NH_2$ (a)

$H_2N\text{-}(CH_3)_2\text{-}X\text{-}(CH_3)_2\text{-}NH_2$ (b)

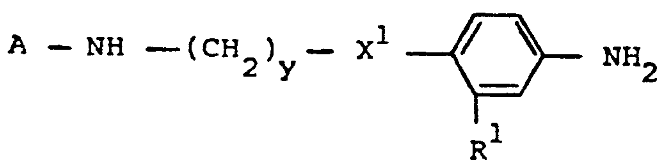

(c)

$H_2N\text{-}(CH_2\text{-}CH_2\text{-}NH)_n\text{-}CH_2\text{-}CH_2\text{-}NH_2$ (d)

in welchen

X ein Sauerstoffatom, ein Schwefelatom, eine Sulfonylgruppe oder eine Gruppe der Formel -NH- , $-N(CH_3)-$ oder $-N(COCH_3)-$ bedeutet,

$R^1$ für ein Wasserstoffatom oder eine Sulfogruppe steht,

A die obengenannte Bedeutung besitzt,

n die Zahl 2, 3 oder 4 ist,

$X^1$ für die Gruppe -NH- oder für ein Sauerstoffatom steht und

y die Zahl 2 oder 3 bedeutet,

des weiteren das 1,3- oder 1,4-Cyclohexylen-diamin, das Bis-(4-amino-cyclohex-1-yl)-methan, das 1,8-Di-(aminomethyl)-naphthalin, das 1,4-Di-(aminomethyl)-benzol, das 1,3-Di-(aminomethyl)-benzol, das N,N'-Bis-(β-aminoethyl)-1,4-piperidin, das 1,4- oder 1,3-Phenylendiamin, das 4-Amino-benzylamin, das 4-Amino-phenethylamin sowie die entsprechenden N-Monoacyl-Derivate solcher Verbindungen. Bevorzugt sind hiervon 1,2-Diamino-ethan, 1,3-Diamino-propan, 1,4-Diamino-butan und 1,2-Diamino-propan.

Da bei der Umsetzung der Verbindungen (4) mit den Verbindungen (5) Halogenwasserstoff abgespalten wird, ist es erforderlich, in Gegenwart eines säurebindenden Mittels zu arbeiten. Hierfür kommen sowohl anorganische Verbindungen, wie beispielsweise Natriumhydroxid, Kaliumhydroxid, Natriumcarbonat, Calciumcarbonat, Magnesiumoxid, Natriumacetat oder Kaliumacetat, als auch organische basische Verbindungen, wie beispielsweise Trialkylamine mit Alkylresten von 1 bis 4 C-Atomen, wie z.B. Trimethylamin und Triethylamin, oder Pyridin, Chinolin, die Picoline und Morpholin, in Frage. Das säurebindende Mittel muß jeweils in mindestens äquivalenter Menge zur Monohalogenverbindung (4) vorhanden sein. Stellt die Aminoverbindung (5) eine ausreichend basisch reagierende Verbindung dar, wie beispielsweise Ethylendiamin, und setzt man diese in ausreichendem Überschuß ein, so wirkt diese Verbindung als Säureakzeptor, wodurch der Zusatz eines gesonderter anorganischen oder organischen säurebindenden Mittels entfällt.

Geeignete Lösemittel, die als Reaktionsmedium bei der Umsetzung von (4) mit (5) dienen können, sind beispielsweise Wasser, Alkanole von 1 bis 4 C-Atomen, wie Methanol, Ethanol, Propanol und Isopropanol, Dioxan, Toluol, die Xylole, Chlorbenzol, o-Dichlorbenzol, m-Dichlorbenzol, Dimethylformamid oder N-Methyl-pyrrolidon. Ist eine der beiden Reaktanten (4) und (5) oder beide in dem angewandten Lösemittel nicht vollständig löslich, so findet die Reaktion teilweise in Suspension statt, was ihr keinen Abbruch tut. Es kann jedoch auch die Verbindung (5) durch Anwendung in einem ausreichenden Überschuß als Lösemittel eingesetzt werden.

Bei der Anwendung eines Lösemittels, in welchem sich die beiden Reaktanten (4) und (5) jeweils vollständig lösen, ist es zweckmäßig, die Verbindungen (4) und (5) im molaren Verhältnis 1:(1 - 2,5) zur Umsetzung zu bringen. Die Anwendung eines größeren Überschusses an der Verbindung (5) ist zwar möglich, bringt aber keinen Vorteil mehr und ist daher nicht zweckmäßig.

Bei Anwendung eines Lösemittels, in welchem sich einer oder beide Reaktanten (4) und (5) nicht vollständig lösen, oder bei Anwendung von überschüssigem (5) als Lösemittel, ist es zweckmäßig, die Verbindung (5) in der 1- bis 4-fachen Gewichtsmenge, bezogen auf die Verbindung (4), mit der Verbindung (4) zur Umsetzung zu bringen. Die Anwendung eines größeren Überschusses an (5) ist auch hier möglich, aber nicht zweckmäßig.

Die synthetisierte Nitroverbindung der allgemeinen Formel (2) kann in einfacher Weise durch Kristallisation aus dieser Reaktionsmischung und anschließende Filtration isoliert werden oder auch, indem man das Lösemittel bzw. das überschüssige Reagenz (5), das vorteilhafterweise für weitere Umsetzungen wiedergewonnen werden kann, gegebenenfalls teilweise oder vollständig abdestilliert und anschließend das eventuell eingeengte Reaktionsgemisch unter Rühren mit kaltem Wasser, daß gegebenenfalls ein Elektrolytsalz, wie Natriumchlorid, Kaliumchlorid oder Natriumsulfat, gelöst enthält, zusammengibt. Das hierbei auskristallisierende Produkt kann durch Filtration, gegebenenfalls nach Ansäuern mit Mineralsäure, isoliert werden.

Die anschließende Reduktion der Nitroverbindung (2) zur entsprechenden Aminoverbindung (3) kann in an sich bekannter Weise durch katalytische Hydrierung mit Wasserstoff an bekannten Katalysatoren, wie Palladium, Platin oder Raney-Nickel, bei einer Temperatur zwischen 20 und 150°C, vorzugsweise zwischen 50 und 110°C, und bei erhöhtem Druck, beispielsweise zwischen 30 und 100 bar, vorzugsweise zwischen 40 und 55 bar, oder durch Reduktion nach Béchamp mit Eisen in saurem oder alkalischem Medium, vorzugsweise in saurem Medium, beispielsweise mit Eisen in Ethanol/Eisessig, durchgeführt werden. Besonders vorteilhaft ist es, die aus der Umsetzung der Verbindungen (4) und (5) erhältliche Nitroverbindung (2) ohne deren Zwischenisolierung direkt der katalytischen Hydrierung zu unterwerfen. Auf diese Weise können Engergie- und Abwasserkosten gesenkt werden. Die Reduktion von (2) zu (3), sei sie katalytisch oder nach Béchamp, wird zweckmäßigerweise in einem geeigneten Lösemittel, wie Wasser, Methanol oder Ethanol, oder in einem Gemisch aus Wasser und Methanol bzw. Wasser und Ethanol durchgeführt, weil sich hierbei die Aminoverbindungen (3) in einfacher Weise durch Abkühlen, gegebenenfalls nach Ansäuern mit Mineralsäure, als freie Aminoverbindungen oder in Form der mineralsauren Salze zur Kristallisation bringen lassen können; gegebenenfalls lassen sie sich auch durch Natriumchlorid aussalzen. Die anschließende Abtrennung erfolgt sodann durch Filtration, und die Mutterlaugen können für nachfolgende Hydrieransätze wieder verwendet werden und hierfür gegebenenfalls im Kreislauf geführt werden. Ebenso läßt sich aus den Mutterlaugen das organische Lösemittel durch einfache Destillation bei Normaldruck zurückgewinnen.

Die erfindungsgemäßen Verfahrensweisen machen es möglich, die neuen Verbindungen der allgemeinen Formel (1) in guten bis sehr guten Ausbeuten zu gewinnen. Sie stellen neue, wertvolle Zwischenprodukte zur Herstellung von faserreaktiven Farbstoffen, insbesondere von faserraktiven Triphendioxazinfarbstoffen, dar, wie beispielsweise zur Herstellung von Farbstoffen der allgemeinen Formel (A)

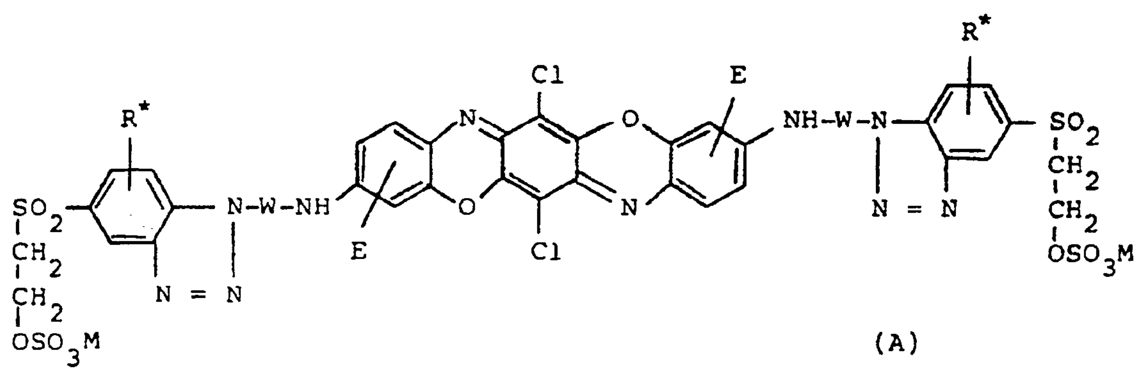

(A)

in welcher W und R* die obengenannten Bedeutungen haben, M ein Alkalimetall und E ein Wasserstoffatom oder eine Sulfo-, Carboxy-, $C_1$-$C_4$-Alkylsulfonyl- oder Sulfo-$C_1$-$C_4$-alkylsulfonyl-Gruppe oder eine β-Sulfatoethylsulfonyl-Gruppe bedeutet. Besonders vorteilhafte Triphendioxazinfarbstoffe entsprechend der allgemeinen Formel (A) sind beispielsweise diejenigen mit E gleich einer Sulfogruppe und W einer Ethylen- oder einer

n-Propylen-Gruppe, die auf Baumwolle nach den in der Technik für faserreaktive Farbstoffe üblichen Färbeweisen Färbungen in reinen blauen und echten Tönen liefern.

Die erfindungsgemäße Verwendung der Verbindungen (1) als Zwischenprodukte zur Synthese von faserreaktiven Farbstoffen erfolgt beispielsweise so, daß man zunächst die Verbindung (1) mit einer Chlornitroverbindung, beispielsweise mit 2-Sulfo-4-nitrochlorbenzol, 2-(β-Sulfoethylsulfonyl)-4-nitrochlorbenzol oder 2-(β-Hydroxyethylsulfonyl)-4-nitrochlorbenzol, zu einer Verbindung der allgemeinen Formel (6)

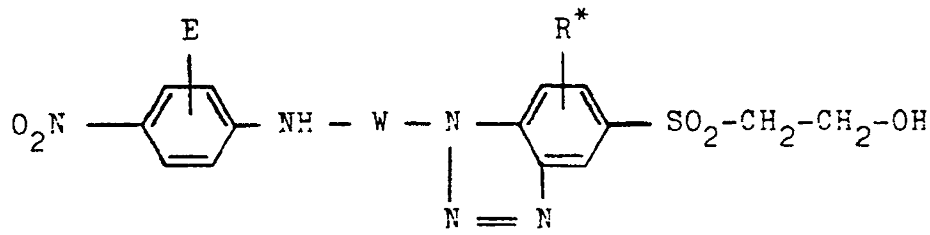

(6)

in welcher W, R* und E die obengennanten Bedeutungen haben oder E auch eine β-Hydroxyethylsulfonyl-Gruppe ist, umsetzt (wobei die Umsetzung der Aminoverbindung mit den Chlorbenzolverbindung analog den obigen Angaben in an und für sich bekannter Verfahrensweise erfolgen kann) und in dieser Verbindung (6) die Nitrogruppe in an und für sich bekannter Verfahrensweise, wie oben beispielsweise beschrieben, zur Aminogruppe in den Verbindungen der allgemeinen Formel (7)

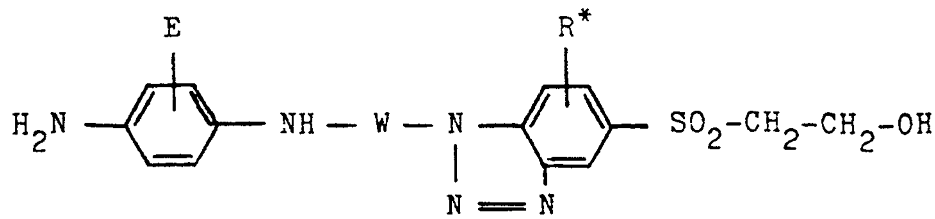

(7)

in welchen W, R* und E die obengenannten Bedeutungen haben, reduziert. Die Aminoverbindung der allgemeinen Formel (7) läßt sich sodann mit 2,3,5,6-Tetrachloro-1,4-benzochinon in an und für sich bekannter Verfahrensweise der Herstellung von faserreaktiven Triphendioxazinverbindungen (s. beispielsweise die Europäische Patentanmeldungs-Veröffentlichung Nr. 0 168 751 Al) unter oxidativem Ringschluß und gleichzeitiger oder nachfolgender Sulfatierung in Schwefeltrioxid enthaltender Schwefelsäure zu den Farbstoffen der Formel (A) umsetzen.

Beispiel 1

a) 530 Teile 2-Nitro-4-(ß-hydroxyethylsulfonyl)-chlorbenzol werden bei einer Temperatur zwischen 70 und 80°C langsam in 620 Teile Ethylendiamin eingetragen; der Ansatz wird einige Zeit bei dieser Temperatur gehalten und sodann, nach quantitativer Umsetzung, in Wasser eingerührt. Die ausgefallene Verbindung der Formel

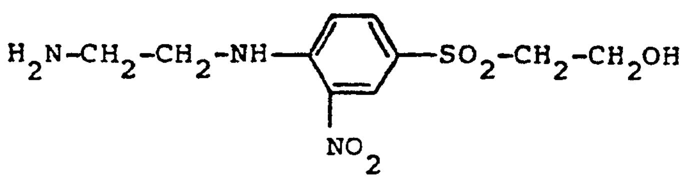

wird durch Filtration isoliert und getrocknet. Sie wird in guter Ausbeute und hoher Reinheit mit einem Fp. 146/147°C erhalten und zeigt in der 1H-NMR-Analyse (in $d_6$-DMSO mit TMS als inneren Standard) folgende Daten:

δ = 2,83 ppm (t,2H); 3,44 ppm (m,2H); 3,7 ppm (m,2H); 7,27 ppm (d,1H); 7,9 ppm (dd,1H); 8,49 ppm (d,1H); bewegliche Protonen bei 1,7 ppm ($NH_2$), 4,8 ppm (OH) und 8,8 ppm (NH).

b) Die unter a) erhaltene Nitroverbindung wird zur Anilinverbindung reduziert, indem man 290 Teile der

Nitroverbindung in 1200 Teilen Wasser in Gegenwart eines Pd/C-Katalysators in einem Auoklaven bei einer Temperatur bis 100°C und einem Wasserstoffdruck von 50 bar hydriert. Der Katalysator wird anschließend abfiltriert; die erhaltene Aminoverbindung kann im Filtrat direkt weiter verarbeitet werden. Eine isolierte Probe der erhaltenen Anilinverbindung der Formel

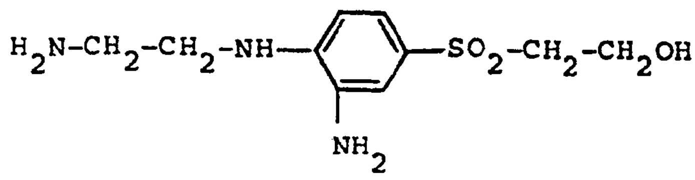

die einen Fp. von 169-172°C aufweist, zeigt folgende Daten in der 1H-NMR-Analyse (in $d_6$-DMSO mit TMS als inneren Standard):

δ = 2,84 ppm (t,2H); 3,2 ppm (m,4H); 3,6 ppm (m,2H); 6,53 ppm (dd,1H); 6,96 ppm (d,1H); 7,0 ppm (dd,1H); mobile Protonen bei 4 - 5 ppm ($NH_2$,OH) und 5,45 ppm (NH).

c) 260 Teile β-[4-(β-Hydroxyethylsulfonyl)-2-amino-phenyl-amino]-ethylamin werden in etwa 1800 Teilen einer salzsauren wäßrigen Lösung bei 0 bis 5°C mittels einer wäßrigen Natriumnitritlösung in üblicher Weise diazotiert. Der Ringschluß erfolgt sofort und quantitativ. Eine isolierte Probe der Verbindung der Formel

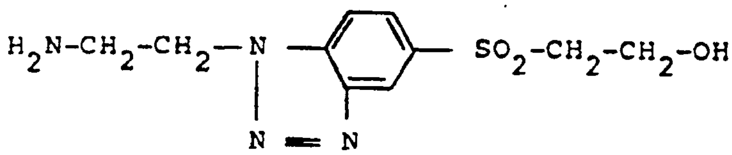

zeigt folgende Daten in der $^1$H-NMR-Analyse (in $d_6$-DMSO mit TMS als inneren Standard):

δ = 3,06 ppm (t,2H); 3,54 ppm (m,2H); 3,7 ppm (m,2H); 4,72 ppm (t,2H); 8,0 ppm (dd,1H); 8,16 ppm (dd,1H); 8,6 ppm (m,1H); mobile Protonen (OH, $NH_2$).

Beispiel 2

a) 530 Teile 2-Nitro-4-(β-hydroxyethylsulfonyl)-chlorbenzol werden bei einer Temperatur zwischen 70 und 80°C langsam in 750 Teile 1,3-Propylendiamin eingetragen; der Ansatz wird einige Zeit bei dieser Temperatur gehalten und sodann, nach quantitativer Umsetzung, in Wasser eingerührt. Die ausgefallene Verbindung der Formel

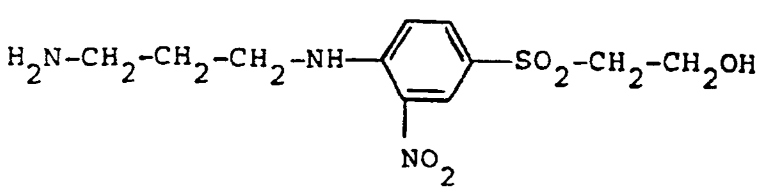

wird durch Filtration isoliert und getrocknet. Sie wird in guter Ausbeute und hoher Reinheit mit einem Fp. 110/112°C erhalten und zeigt in der 1H-NMR-Analyse (in $d_6$-DMSO mit TMS als inneren Standard) folgende Daten:

δ = 1,7 ppm (m,2H); 2,66 ppm (t,2H); 3,5 ppm (m,4H); 3,68 ppm (m,2H); 7,25 ppm (d,1H); 7,9 ppm (dd,1H); 8,5 ppm (d,1H); mobile Protonen ($NH_2$, OH, NH) bei ca. 5 ppm.

b) Die unter a) erhaltene Nitroverbindung wird zur Anilinverbindung reduziert, indem man 303 Teile der Nitroverbindung in 1200 Teilen Wasser in Gegenwart eines Pd/C-Katalysators in einem Auoklaven bei einer Temperatur bis 100°C und einem Wasserstoffdruck von 50 bar hydriert. Der Katalysator wird anschließend abfiltriert; die erhaltene Aminoverbindung kann im Filtrat direkt weiter verarbeitet werden. Eine isolierte Probe der erhaltenen Anilinverbindung der Formel

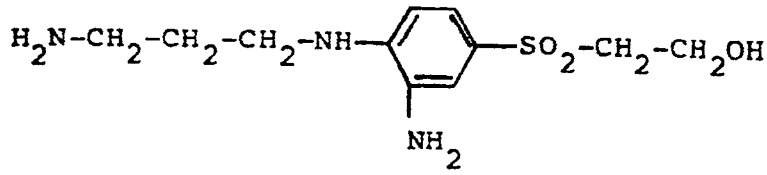

zeigt folgende Daten in der 1H-NMR-Analyse (in $d_6$-DMSO mit TMS als inneren Standard):

δ = 1,9 ppm (m,2H); 2,88 ppm (t,2H); 3,2 ppm (m,4H); 3,58 ppm (m,2H); 6,51 ppm (dd,1H); 6,92 ppm (d,1H); 6,96 ppm (dd,1H); mobile Protonen bei 4,9 ppm (OH), 5,2 ppm ($NH_2$), 5,74 ppm (NH) und 8,2 ppm ($NH_2$).

c) 260 Teile γ-[4-(β-Hydroxyethylsulfonyl)-2-amino-phenyl-amino]-n-propylamin werden in etwa 1800 Teilen einer salzsauren wäßrigen Lösung bei 0 bis 5°C mittels einer wäßrigen Natriumnitritlösung in üblicher Weise diazotiert. Der Ringschluß erfolgt sofort und quantitativ. Eine isolierte Probe der Verbindung der Formel

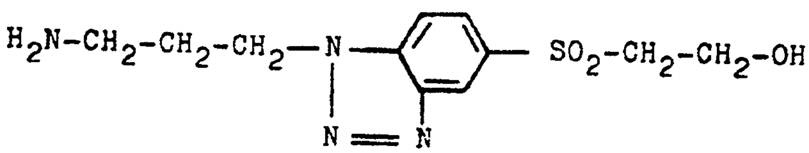

zeigt folgende Daten in der $^1$H-NMR-Analyse (in $d_6$-DMSO mit TMS als inneren Standard):

=2,24 ppm (m,2H); 2,85 ppm (m,2H); 3,6 ppm (m,4H); 4,92 ppm (t,2H); 8,04 ppm (dd,1H); 8,27 ppm (d,1H); 8,6 ppm (s,1H); mobile Protonen (OH, $NH_2$).

Beispiel 3

Zu einer Lösung von 118,8 Teilen 1,4-Phenylendiamin in 500 Volumenteilen Methanol werden bei 65°C unter Stickstoffatmosphäre 132,8 Teile 4-(β-Hydroxyethylsulfonyl)-2-nitro-chlorbenzol innerhalb von 30 Minuten eingetragen. Man rührt noch 4 Stunden bei dieser Temperatur nach, läßt auf Raumtemperatur abkühlen und das Reaktionsprodukt durch Zugabe von 1000 Volumenteilen Eiswasser auskristallisieren. Nach Absaugen und Trocknen erhält man das 4-(β-Hydroxyethylsulfonyl)-2-nitro-4'-amino-diphenylamin in hoher Ausbeute. Es wird mit der äquimolaren Menge an Essigsäureanhydrid, beispielsweise in Eisessig, bei einer Temperatur von 50°C N-acyliert; die Acetaminoverbindung, die einen Fp. von 193/194°C besitzt, wird anschließend zur Anilinoverbindung der Formel

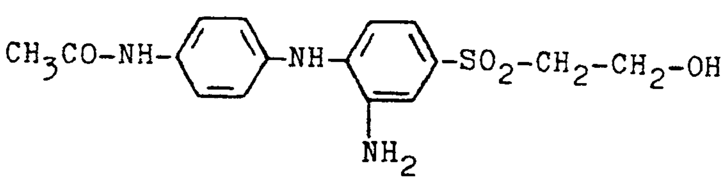

(Fp.: 183/184°C)

reduziert, die duch Diazotierung und anschließende saure Entacylierung gemäß üblichen Verfahrensweisen in die erfindungsgemäße Verbindung der Formel

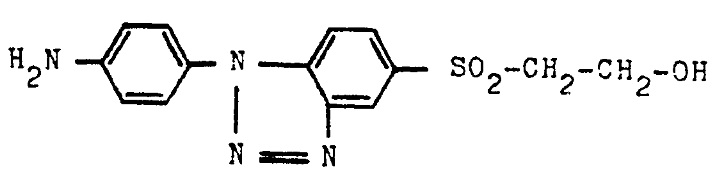

übergeführt wird. Die erfindungsgemäße Verbindung besitzt einen Schmelzpunkt von 177°C. In der 1H-NMR-Analyse (in $d_6$-DMSO mit TMS als innerem standard) zeigt sie folgende Daten:

δ = 3,6 ppm (t,2H); 3,8 ppm (m,2H); 4,9 ppm (t, OH); 5,7 ppm (s,$NH_2$); 6,8 ppm (d,2H); 7,5 ppm (d, 2H); 8,0 ppm (d,1H); 8,1 ppm (d,1H); 8,7 ppm (s,1H).

Anstelle von 1,4-Phenylendiamin als Ausgangsverbindung kann bevorzugt auch das 4-Amino-acetanilid eingesetzt werden. Hierbei verwendet man als säurebindendes Mittel beispielsweise Natriumacetat, Triethylamin oder Triethanolamin; die anschließende N-Acetylierung erübrigt sich demgemäß.

Beispiel 4

Man verfährt zur Herstellung, einer erfindungsgemäßen Verbindung gemäß der Verfahrensweise des Beispieles 3, setzt jedoch anstelle von 1,4-Phenylendiamin die gleiche Menge an 1,3-Phenylendiamin bzw. bevorzugt 3-Amino-acetanilid ein. Man erhält auf diese Weise die erfindungsgemäße Verbindung der formel

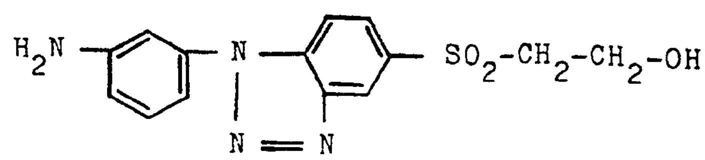

Anwendungsbeispiel 1

Die in Beispiel 1c) beschriebene erfindungsgemäße Verbindung kann wie folgt zu einem Farbstoff weiterverarbeitet werden:

a) Eine Mischung von 270 Teilen 5-(β-Hydroxyethylsulfonyl)-1-(β-aminoethyl)-benzotriazol in 1000 Teilen Wasser und 500 Teilen Triethanolamin werden zunächst bei einer Temperatur von 40°C mit 259 Teilen des Natriumsalzes der 5-Nitro-2-chlorbenzolsulfonsäure versetzt (die Benzotriazolverbindung kann auch in Form der gemäß Beispiel 1c) erhaltenen Lösung direkt eingesetzt werden). Innerhalb von zwei Stunden wird die Mischung auf 100 bis 110°C aufgeheizt, wobei das Wasser teilweise abdestilliert. Man rührt weitere 10 Stunden bei 115 bis 120°C zur quantitativen Umsetzung nach und gibt sodann bei 100°C 3000 Teile Wasser hinzu und klärt die Lösung bei 80 bis 90°C. Aus dem wäßrigen Medium kristallisiert beim Abkühlen das Natriumsalz des 4-[β-(5′-β′-Hydroxyethylsulfonylbenzotriazol-1′-yl)-ethylamino]-3-sulfo-nitrobenzol in hoher Ausbeute und Reinheit aus. Die Nitroverbindung zeigt folgende Daten in der $^1$H-NMR-Analyse (in $D_6$-DMSO mit TMS als innerem Standard):
δ = 3,5 ppm (m,2H); 3,64 ppm (t,2H); 3,95 ppm (m,2H); 4,82 ppm (t,OH); 5,0 ppm (t,2H); 6,69 ppm (d,1H); 7,57 ppm (t,NH); 7,95 ppm (m,2H); 8,16 ppm (m,1H); 8,3 ppm (d,1H); 8,57 ppm (d,1H).
Die Nitroverbindung wird anschließend durch katalysische Hydrierung zur Anilinverbindung reduziert, indem man 236 Teile der Nitroverbindung in 1000 Teilen Wasser löst und in Gegenwart eines Pd/C-Katalysators in einem Autoklaven bei einer Temperatur bis 100°C und einem Wasserstoffdruck von 50 bar hydriert. Der Katalysator wird anschließend abfiltriert, das Filtrat abgekühlt und angesäuert. Aus ihm kristallisiert die Anilinverbindung der Formel

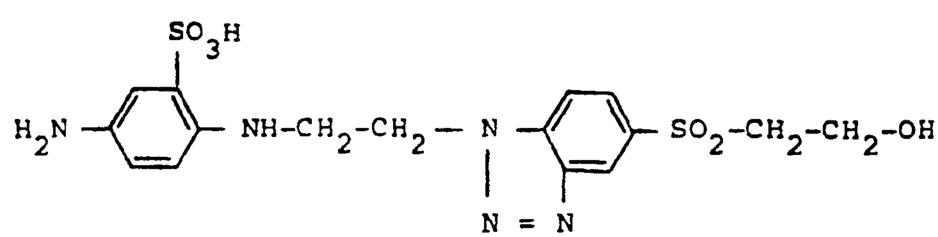

in guter Ausbeute und hoher Reinheit aus.

b) 441 Teile der unter a) erhaltenen Anilinverbindung werden in 2000 Teilen Wasser bei pH 6 und 60°C gelöst. 124 Teile Chloranil werden eingetragen, wobei mit etwa 90 Teilen Natriumbicarbonat ein pH-Wert von 6,5 und eine Reaktionstemperatur von etwa 65°C gehalten wird. Der Ansatz wird noch sechs Stunden nachgerührt, das Reaktionsprodukt sodann bei etwa 65°C geklärt, mit wenig Natriumchlorid ausgefällt, abgesaugt, mit 1000 Teilen einer 10%igen wäßrigen Natriumchloridlösung gewaschen und unter reduziertem Druck bei 70°C getrocknet.

c) 105 Teile des unter b) erhaltenen Produktes werden bei einer Temperatur zwischen 20 und 25°C in 750 Teile 13%iges Oleum eingetragen. Der Reaktionsansatz wird anschließend noch etwa drei Stunden bei

dieser Temperatur nachgerührt; sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, filtriert die ausgefallene erfindungsgemäße Verbindung ab, löst sie wieder in etwa 1000 Teilen Wasser, stellt mit Natriumcarbonat einen pH-Wert von 5 ein und salzt sie, gegebenenfalls nach vorheriger üblicher Klärung der Lösung, mit Natriumchlorid aus.

Die erfindungsgemäße Triphendioxazin-Verbindung kann auch in Form ihres Natriumsalzes durch Eindampfen oder Sprühtrocknen der geklärten Syntheselösung erhalten werden. Sie besitzt, in Form der freien Säure geschrieben, die mutmaßliche Formel

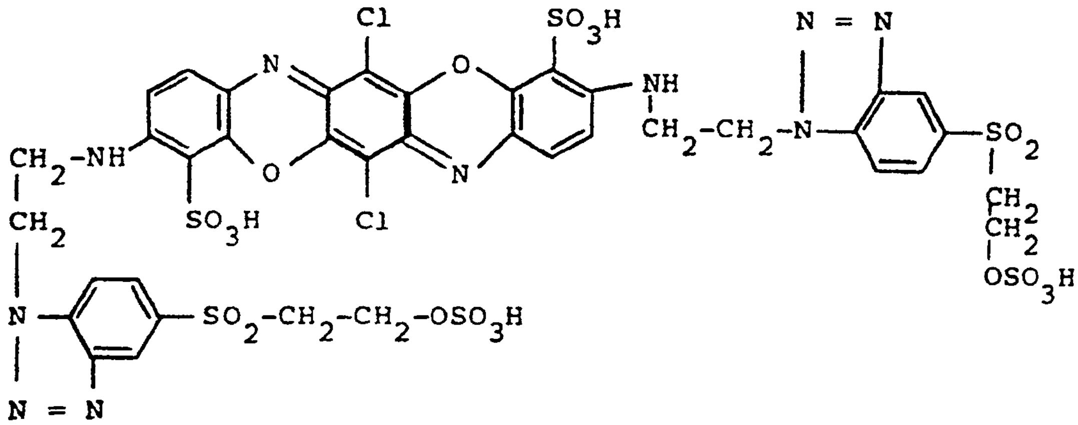

(die jeweilige Sulfogruppe kann auch in der anderen ortho-Stellung zur Benztriazolyl-ethylamino-Gruppe gebunden sein, befindet sich jedoch mit größerer Wahrscheinlichkeit in der in der obigen Formel angegebenen Stellung).

Diese erfindungsgemäße Verbindung besitzt gute faserreaktive Farbstoffeigenschaften. Sie färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen rotstichig blauen Tönen (entsprechend der Farbkennzahl 13 von Colour Index Hue Indication Chart) mit guten Echtheiten, wie insbesondere guter Lichtechtheit der trockenen oder feuchten, wie mit Trinkwasser befeuchteten Färbung, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit.

In wäßriger Lösung zeigt sie im sichtbaren Bereich ein Absorptionsmaximum bei 612 nm.

Anwendungsbeispiel 2

Die in Beispiel 2c) beschriebene erfindungsgemäße Verbindung kann wie folgt zu einem Triphendioxazinfarbstoff weiterverarbeitet werden:

Eine gemäß Beispiel 2c) erhaltene wäßrig-saure Lösung mit 285 Teilen 5-(β-Hydroxyethylsulfonyl)-1-(γ-aminopropyl)benzotriazol und 500 Teilen Triethanolamin wird auf 40°C erwärmt und mit 259 Teilen des Natriumsalzes der 5-Nitro-2-chlorbenzolsulfonsäure versetzt. Innerhalb von zwei Stunden wird die Mischung auf 100 bis 110°C aufgeheizt, wobei das Wasser teilweise abdestilliert. Man rührt weitere 10 Stunden bei 115 bis 120°C zur quantitativen Umsetzung nach und gibt sodann bei 100°C 3000 Teile Wasser hinzu und klärt die Lösung bei 80 bis 90°C. Aus dem wäßrigen Medium kristallisiert beim Abkühlen das Natriumsalz des 4-[γ-(5′-β′-Hydroxyethylsulfonylbenzotriazol-1′-yl)-propylamino]-3-sulfo-nitrobenzol in hoher Ausbeute und Reinheit aus. Die Nitroverbindung zeigt folgende Daten in der $^1$H-NMR-Analyse (in $d_6$-DMSO mit TMS als innerem Standard):

δ = 2,25 ppm (m,2H); 3,32 ppm (m,2H); 3,54 ppm (m,2H); 3,66 ppm (m,2H); 4,9 ppm (m,OH,2H); 6,9 ppm (d,1H); 7,47 ppm (t,NH); 8,0 ppm (m,2H); 8,20 ppm (m,1H); 8,37 ppm (d,1H); 8,62 ppm (d,1H).

Die so erhaltene Nitroverbindung kann sodann gemäß den Angaben des Beispieles 1a) zur Anilinverbindung der Formel

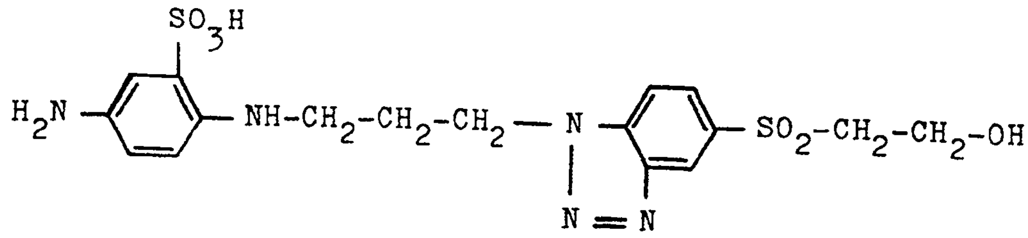

reduziert und sodann gemäß den Angaben des Anwendungsbeispieles 1b) und 1c) zu einem wertvollen blauen Triphendioxazinfarbstoff umgesetzt werden.

## Patentansprüche

### Patentansprüche für folgende Vertragsstaaten : BE, CH, DE, FR, GB, IT, LI

1. Verbindung entsprechend der allgemeinen Formel (1)

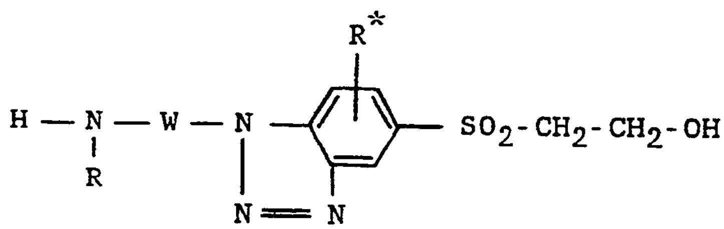
(1)

in welcher
R ein Wasserstoffatom oder eine Alkylgruppe von 1 bis 6 C-Atomen ist, die durch einen oder zwei Substituenten aus der Gruppe Hydroxy, Sulfo, Carboxy, Phosphono und Cyano substituiert sein kann,
W den 1,2-Ethylen-, 1,3-Propylen-, 1,4-Butylen-, 1,5-Pentylen-, 1,6-Hexylen-, 1,2-Propylen-, 1,2-Butylen-, 1,3-Butylen-, 2-Methyl-1,3-propylen-, 2-Hydroxy-1,3-propylen-, 2-Carboxy-1,5-pentylen- oder den 2-Phenyl-1,3-propylen-Rest bedeutet oder ein im Benzolrest sulfosubstituierter 2-Phenyl-1,3-propylen-Rest ist oder einen Rest der allgemeinen Formel (1a), (1b), (1c) oder (1d)

$$-(CH_2)_2-X-(CH_2)_2- \qquad (1a)$$
$$-(CH_2)_3-X-(CH_2)_3- \qquad (1b)$$

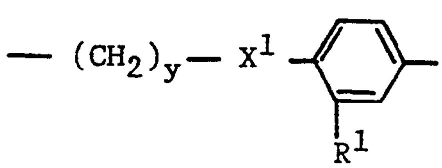
(1c)

$$-(CH_2-CH_2-NH)_n-CH_2-CH_2- \qquad (1d)$$

ist, in welchen
X ein Sauerstoffatom, ein Schwefelatom, eine Sulfonylgruppe oder eine Gruppe der Formel -NH- , $-N(CH_3)-$ oder $-N(COCH_3)-$ bedeutet,
$R^1$ für ein Wasserstoffatom oder eine Sulfogruppe steht,
n die Zahl 2, 3 oder 4 ist,
$X^1$ für die Gruppe -NH- oder für ein Sauerstoffatom steht und
y die Zahl 2 oder 3 bedeutet,
oder der 1,3-Cyclohexylen-, 1,4-Cyclohexylen-, Bis-(1,4-cyclohexylen)-methan-, 1,8-Di-methylennaphthalin-, 1,4-Di-methylen-benzol-, 1,3-Di-methylenbenzol-, 1,4-Bis-(1′,2′-ethylen)-piperidin-, 1,4-Phenylen- oder 1,3-Phenylen-Rest oder ein Rest der allgemeinen Formel (1e) oder (1f)

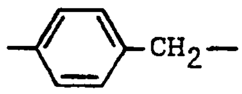 (1e)

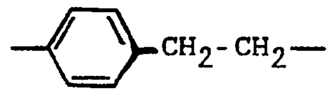 (1f)

ist, und
R* ein Wasserstoffatom oder eine Alkylgruppe von 1 bis 6 C-Atomen, eine Alkoxygruppe von 1 bis 5 C-Atomen, ein Halogenatom, eine Carboxygruppe oder eine Sulfogruppe bedeutet.

2. Verbindung nach Anspruch 1, dadurch gekennzeichnet, daß R* ein Wasserstoffatom ist.

3. Verbindung nach Anspruch 1 oder 2, dadurch gekennzeichnet, daß R ein Wasserstoffatom ist.

4. Verbindung nach Anspruch 1, 2 oder 3, dadurch gekennzeichnet, daß W ein para- oder ein meta-Phenylenrest ist.

5. Verbindung nach Anspruch 1, 2 oder 3, dadurch gekennzeichnet, daß W ein 1,2-Ethylen-, 1,3-Propylen-, 1,4-Butylen- oder ein Isopropylen-Rest ist.

6. Verfahren zur Herstellung einer in Anspruch 1 genannten und definierten Verbindung der allgemeinen Formel (1), dadurch gekennzeichnet, daß man in einer Verbindung der allgemeinen Formel (2)

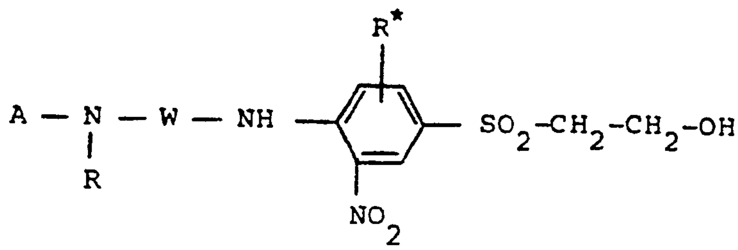 (2)

in welcher R, R* und W die in Anspruch 1 genannten Bedeutungen haben und A ein Wasserstoffatom oder ein Acylrest ist, wobei im Falle, daß W einen aromatisch-carbocyclischen Rest bedeutet, A zwingend einen Acylrest darstellt, deren Nitrogruppe zur Aminogruppe reduziert und die Verbindung der allgemeinen Formel (3)

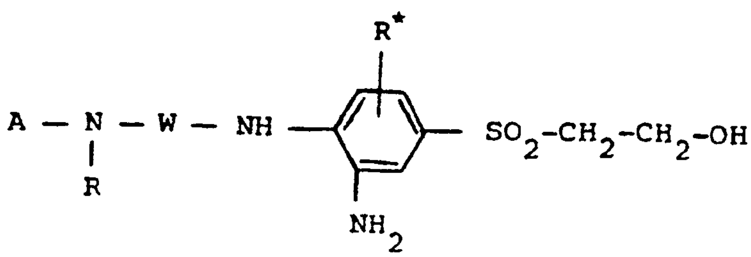 (3)

in welcher A, R, R* und W die in Anspruch 1 genannten Bedeutungen haben, diazotiert und gegebenenfalls im Falle, daß in Formel (3) die Gruppe A einen Acylrest bedeutete, diese Acylbenztriazolverbindung zur Benztriazolverbindung der allgemeinen Formel (1) entacyliert.

7. Verwendung einer Verbindung von mindestens einem der Ansprüche 1 bis 6 zur Synthese von Farbstoffen.

8. Verwendung nach Anspruch 7 zur Synthese von faserreaktiven Triphendioxazinfarbstoffen.

**Patentansprüche für folgenden Vertragsstaat : ES**

1. Verfahren zur Herstellung einer Verbindung entsprechend der allgemeinen Formel (1)

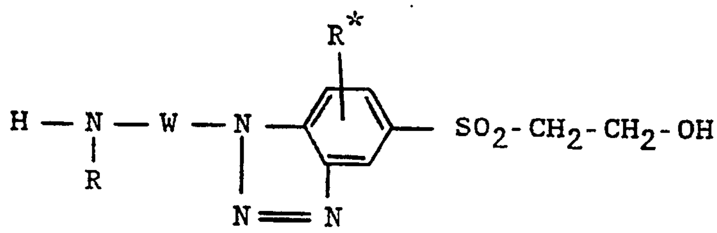

(1)

in welcher
R ein Wasserstoffatom oder eine Alkylgruppe von 1 bis 6 C-Atomen ist, die durch einen oder zwei Substituenten aus der Gruppe Hydroxy, Sulfo, Carboxy, Phosphono und Cyano substituiert sein kann,
W den 1,2-Ethylen-, 1,3-Propylen-, 1,4-Butylen-, 1,5-Pentylen-, 1,6-Hexylen-, 1,2-Propylen-, 1,2-Butylen-, 1,3-Butylen-, 2-Methyl-1,3-propylen-, 2-Hydroxy-1,3-propylen-, 2-Carboxy-1,5-pentylen- oder den 2-Phenyl-1,3-propylen-Rest bedeutet oder ein im Benzolrest sulfosubstituierter 2-Phenyl-1,3-propylen-Rest ist oder einen Rest der allgemeinen Formel (1a), (1b), (1c) oder (1d)

$-(CH_2)_2-X-(CH_2)_2-$ (1a)

$-(CH_2)_3-X-(CH_2)_3-$ (1b)

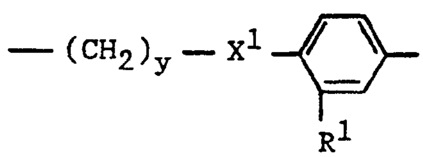

(1c)

$-(CH_2-CH_2-NH)_n-CH_2-CH_2-$ (1d)

ist, in welchen
X ein Sauerstoffatom, ein Schwefelatom, eine Sulfonylgruppe oder eine Gruppe der Formel -NH- , $-N(CH_3)-$ oder $-N(COCH_3)-$ bedeutet,
$R^1$ für ein Wasserstoffatom oder eine Sulfogruppe steht,
n die Zahl 2, 3 oder 4 ist,
$X^1$ für die Gruppe -NH- oder für ein Sauerstoffatom steht und
y die Zahl 2 oder 3 bedeutet,
oder der 1,3-Cyclohexylen-, 1,4-Cyclohexylen-, Bis-(1,4-cyclohexylen)-methan-, 1,8-Di-methylennaphthalin-, 1,4-Di-methylen-benzol-, 1,3-Di-methylenbenzol-, 1,4-Bis-(1′,2′-ethylen)-piperidin-, 1,4-Phenylen- oder 1,3-Phenylen-Rest oder ein Rest der allgemeinen Formel (1e) oder (1f)

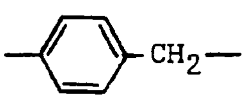

(1e)

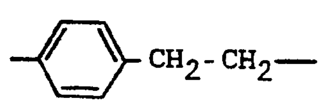

(1f)

ist, und
R* ein Wasserstoffatom oder eine Alkylgruppe von 1 bis 6 C-Atomen, eine Alkoxygruppe von 1 bis 5 C-Atomen, ein Halogenatom, eine Carboxygruppe oder eine Sulfogruppe bedeutet,
dadurch gekennzeichnet, daß man in einer Verbindung der allgemeinen Formel (2)

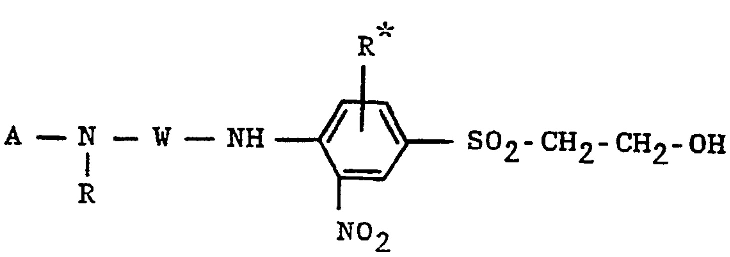

(2)

in welcher R, R* und W die oben genannten Bedeutungen haben und A ein Wasserstoffatom oder ein Acylrest

ist, wobei im Falle, daß W einen aromatisch-carbocyclischen Rest bedeutet, A zwingend einen Acylrest darstellt, deren Nitrogruppe zur Aminogruppe reduziert und die Verbindung der allgemeinen Formel (3)

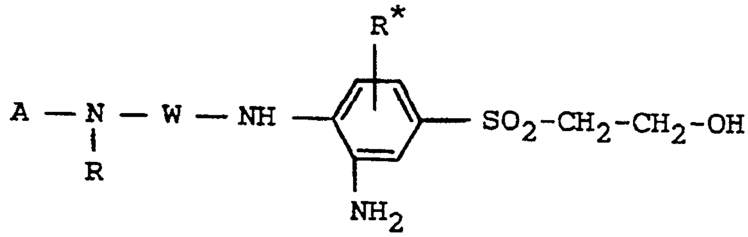

(3)

in welcher A, R, R* und W die oben genannten Bedeutungen haben, diazotiert und gegebenenfalls im Falle, daß in Formel (3) die Gruppe A einen Acylrest bedeutete, diese Acylbenztriazolverbindung zur Benztriazolverbindung der allgemeinen Formel (1) entacyliert.

2. Verfahren nach Anspruch 1, dadurch gekennzeichnet, daß R* ein Wasserstoffatom ist.

3. Verfahren nach Anspruch 1 oder 2, dadurch gekennzeichnet, daß R ein Wasserstoffatom ist.

4. Verfahren nach mindestens einem der Ansprüche 1 bis 3, dadurch gekennzeichnet, daß W ein para- oder ein meta-Phenylenrest ist.

5. Verfahren nach mindestens einem der Ansprüche 1 bis 3, dadurch gekennzeichnet, daß W ein 1,2-Ethylen-, 1,3-Propylen-, 1,4-Butylen- oder ein Isopropylen-Rest ist.

6. Verwendung einer Verbindung von mindestens einem der Ansprüche 1 bis 5 zur Synthese von Farbstoffen.

7. Verwendung nach Anspruch 6 zur Synthese von faserreaktiven Triphendioxazinfarbstoffen.

## Claims

### Claims for the following Contracting States : BE, CH, DE, FR, GB, IT, LI

1. A compound corresponding to the formula (1)

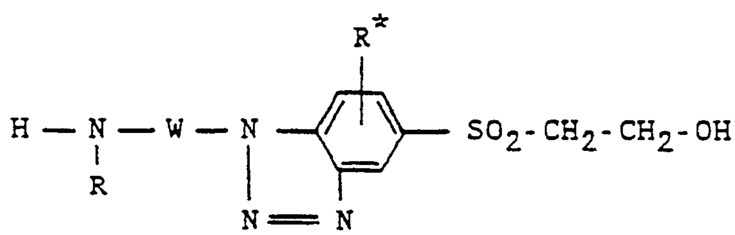

(1)

in which
R is a hydrogen atom or an alkyl group which has 1 to 6 carbon atoms and can be substituted by one or two substituents belonging to the group comprising hydroxyl, sulfo, carboxyl, phosphono and cyano,
W denotes a 1,2-ethylene, 1,3-propylene, 1,4-butylene, 1,5-pentylene, 1,6-hexylene, 1,2-propylene, 1,2-butylene, 1,3-butylene, 2-methyl-1,3-propylene, 2-hydroxy-1,3-propylene, 2-carboxy-1,5-pentylene or a 2-phenyl-1,3-propylene radical or a 2-phenyl-1,3-propylene radical sulfo-substituted in the benzene radical,or a radical of the general formula (1a), (1b), (1c) or (1d)

$$-(CH_2)_2\text{-}X\text{-}(CH_2)_2- \qquad (1a)$$

$$-(CH_2)_3\text{-}X\text{-}(CH_2)_3- \qquad (1b)$$

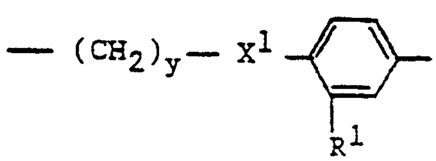

(1c)

$$-(CH_2\text{-}CH_2\text{-}NH)_n\text{-}CH_2\text{-}CH_2- \qquad (1d)$$

in which

X denotes an oxygen atom, a sulfur atom, a sulfonyl group or a group of the formula -NH-, $-N(CH_3)-$ or $-N(COCH_3)-$,

$R^1$ denotes a hydrogen atom or a sulfo group,

n is the number 2, 3 or 4,

$X^1$ denotes the group -NH- or an oxygen atom and

y denotes the number 2 or 3,

or W is a 1,3-cyclohexylene, 1,4-cyclohexylene, bis(1,4-cyclohexylene)methane, 1,8-dimethylenenaphthalene, 1,4-dimethylenebenzene, 1,3-dimethylenebenzene, 1,4-bis(1',2'-ethylene)piperidine, 1,4-phenylene or 1,3-phenylene radical or a radical of the general formula (1e) or (1f)

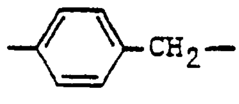 (1e) 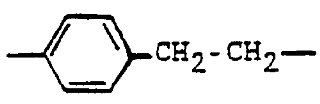 (1f)

and

R* denotes a hydrogen atom or an alkyl group having 1 to 6 carbon atoms, an alkoxy group having 1 to 5 carbon atoms, a halogen atom, a carboxyl group or a sulfo group.

2. A compound as claimed in claim 1, wherein R* is a hydrogen atom.

3. A compound as claimed in claim 1 or 2, wherein R is a hydrogen atom.

4. A compound as claimed in any of claims 1, 2 or 3, wherein W is a para-phenylene or meta-phenylene radical.

5. A compound as claimed in any of claims 1, 2 or 3, wherein W is a 1,2-ethylene, 1,3-propylene, 1,4-butylene or isopropylene radical.

6. A process for the preparation of a compound, mentioned and defined in claim 1, of the formula (1), which comprises reducing the nitro group in a compound of the formula (2)

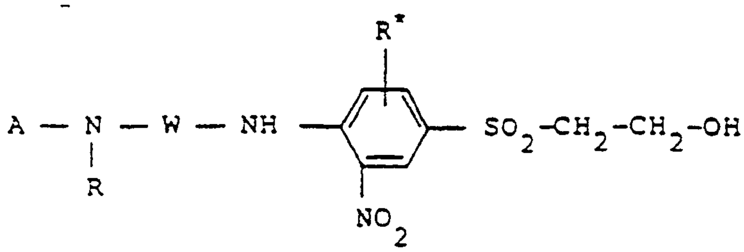 (2)

in which R, R* and W have the meanings mentioned in claim 1 and A is a hydrogen atom or an acyl radical, it being necessary for A to represent an acyl radical in the event that W denotes an aromatic-carbocyclic radical, to the amino group, and diazotizing the compound of the formula (3)

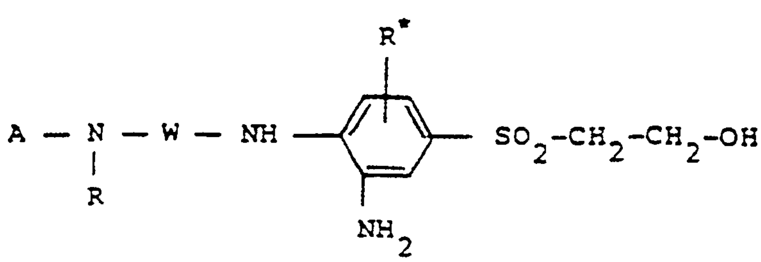 (3)

in which A, R, R* and W have the meanings mentioned in claim 1, and, if appropriate, in the event that the group A in formula (3) should denote an acyl radical, deacylating this acylbenzotriazole compound to give the benzotriazole compound of the formula (1).

7. Use of a compound as claimed in at least one of claims 1 to 6 for the synthesis of dyestuffs.

8. Use as claimed in claim 7 for the synthesis of fiber-reactive triphendioxazine dyestuffs.

**Claims for the following Contracting States : ES**

1. A process for the preparation of a compound corresponding to the formula (1)

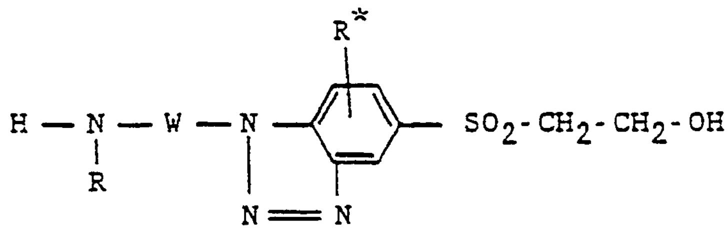
(1)

in which

R is a hydrogen atom or an alkyl group which has 1 to 6 carbon atoms and can be substituted by one or two substituents belonging to the group comprising hydroxyl, sulfo, carboxyl, phosphono and cyano,

W denotes a 1,2-ethylene, 1,3-propylene, 1,4-butylene, 1,5-pentylene, 1,6-hexylene, 1,2-propylene, 1,2-butylene, 1,3-butylene, 2-methyl-1,3-propylene, 2-hydroxy-1,3-propylene, 2-carboxy-1,5-pentylene or a 2-phenyl-1,3-propylene radical or a 2-phenyl-1,3-propylene radical sulfo-substituted in the benzene radical,or a radical of the general formula (1a), (1b), (1c) or (1d)

$$-(CH_2)_2-X-(CH_2)_2- \quad (1a)$$
$$-(CH_2)_3-X-(CH_2)_3- \quad (1b)$$

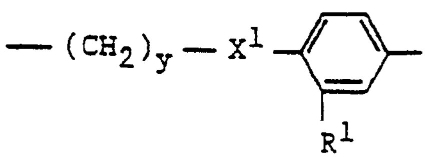
(1c)

$$-(CH_2-CH_2-NH)_n-CH_2-CH_2- \quad (1d)$$

in which

X denotes an oxygen atom, a sulfur atom, a sulfonyl group or a group of the formula -NH-, $-N(CH_3)-$ or $-N(COCH_3)-$,

$R^1$ denotes a hydrogen atom or a sulfo group,

n is the number 2, 3 or 4,

$X^1$ denotes the group -NH- or an oxygen atom and

y denotes the number 2 or 3,

or W is a 1,3-cyclohexylene, 1,4-cyclohexylene, bis(1,4-cyclohexylene)methane, 1,8-dimethylenenaphthalene, 1,4-dimethylenebenzene, 1,3-dimethylenebenzene, 1,4-bis(1',2'-ethylene)piperidine, 1,4-phenylene or 1,3-phenylene radical or a radical of the general formula (1e) or (1f)

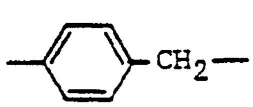
(1e)
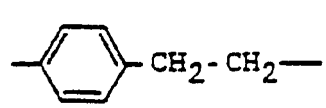
(1f)

and

$R^*$ denotes a hydrogen atom or an alkyl group having 1 to 6 carbon atoms, an alkoxy group having 1 to 5 carbon atoms, a halogen atom, a carboxyl group or a sulfo group,

which comprises reducing the nitro group in a compound of the formula (2)

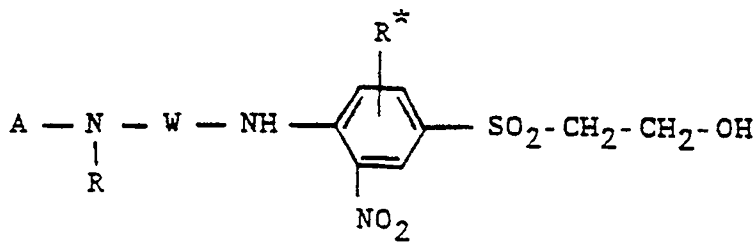 (2)

in which R, R* and W have the meanings mentioned above and A is a hydrogen atom or an acyl radical, it being necessary for A to represent an acyl radical in the event that W denotes an aromaticcarbocyclic radical, to the amino group, and diazotizing the compound of the formula (3)

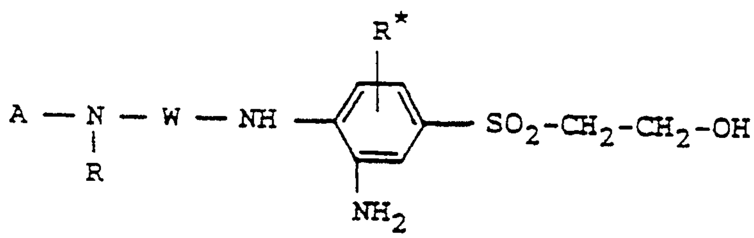 (3)

in which A, R, R* and W have the meanings mentioned above, and, if appropriate, in the event that the group A in formula (3) should denote an acyl radical, deacylating this acylbenzotriazole compound to give the benzotriazole compound of the formula (1).

2. The process as claimed in claim 1, wherein R* is a hydrogen atom.

3. The process as claimed in claim 1 or 2, wherein R is a hydrogen atom.

4. The process as claimed in any of claims 1, 2 or 3, wherein W is a para-phenylene or meta-phenylene radical.

5. The process as claimed in any of claims 1, 2 or 3, wherein W is a 1,2-ethylene, 1,3-propylene, 1,4-butylene or isopropylene radical.

6. Use of a compound of at least one of claims 1 to 5 for the synthesis of dyestuffs.

7. The use as claimed in claim 6 for the synthesis of fiber-reactive triphendioxazine dyestuffs.

**Revendications**

**Revendications pour les Etats contractants suivants : BE, CH, DE, FR, GB, IT, LI**

1. Composé répondant à la formule générale (1) :

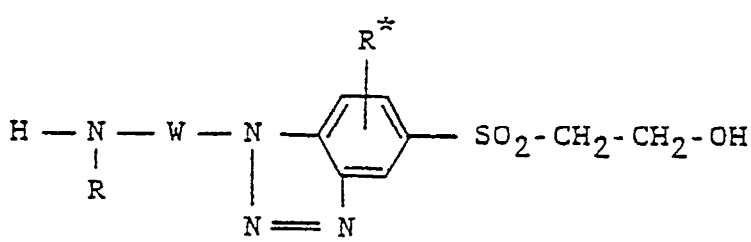 (1)

dans laquelle :

R représente un atome d'hydrogène ou un groupe alkyle ayant 1 à 6 atomes de carbone, qui peut être substitué par un ou deux substituants choisis parmi un groupe hydroxy, sulfo, carboxy, phosphono et cyano,

W représente le reste éthylène-1,2, propylène-1,3, butylène-1,4, pentylène-1,5, hexylène-1,6, propylène-1,2, butylène-1,2, butylène-1,3, méthyl-2 propylène-1,3, hydroxy-2 propylène-1,3, carboxy-2 pentylène-1,5 phényl-2 propylène-1,3, ou W représente un reste phényl-2 propylène-1,3 à substituants sulfo sur le reste benzénique, ou bien W représente un reste répondant à l'une des formules générales (1a), (1b), (1c) ou (1d)

$$—(CH_2)_2\text{-}X\text{-}(CH_2)_2— \quad (1a)$$

$$—(CH_2)_3\text{-}X\text{-}(CH_2)_3— \quad (1b)$$

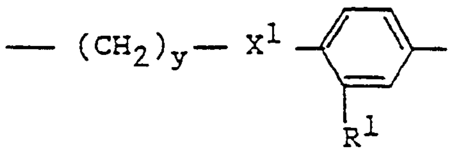

(1c)

$$—(CH_2\text{-}CH_2\text{-}NH)_n\text{-}CH_2\text{-}CH_2— \quad (1d)$$

dans lesquelles :
X représente un atome d'oxygène, un atome de soufre, un groupe sulfonyle ou un groupe de formule -NH-, $(N(CH_3)$- ou $-N(COCH_3)$-,
$R^1$ représente un atome d'hydrogène ou un groupe sulfo,
n est un nombre valant 2, 3 ou 4,
$X^1$ représente le groupe -NH- ou un atome d'oxygène, et
y est un nombre valant 2 ou 3,
ou bien W représente le reste cyclohexylène -1,3, cyclohexylène-1,4, bis-(cyclohexylène-1,4)-méthane, diméthylène-1,8-naphtalène, di-méthylène-1,4 benzène, di-méthylène-1,3 benzène, bis-(éthylène-1',2')-1,4 pipéridine, phénylène-1,4 ou phénylène-1,3, ou un reste de formule générale (1e) ou (1f) :

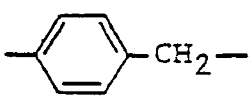

(1e)

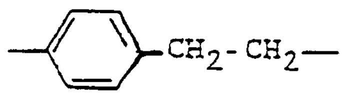

(1f)

et
R* représente un atome d'hydrogène ou un groupe alkyle ayant 1 à 6 atomes de carbone, un groupe alcoxy ayant 1 à 5 atomes de carbone, un atome d'halogène, un groupe carboxyle ou un groupe sulfo.

2. Composé selon la revendication 1, caractérisé en ce que R* représente un atome d'hydrogène.

3. Composé selon la revendication 1 ou 2, caractérisé en ce que R représente un atome d'hydrogène.

4. Composé selon la revendication 1, 2 ou 3, caractérisé en ce que W représente un reste para- ou métaphénylène.

5. Composé selon la revendication 1, 2 ou 3, caractérisé en ce que W représente un reste éthylène-1,2, propylène-1,3, butylène-1,4 ou isopropylène.

6. Procédé pour préparer un composé de formule générale (1), cité et défini à la revendication 1, procédé caractérisé en ce que, dans un composé de formule générale (2) :

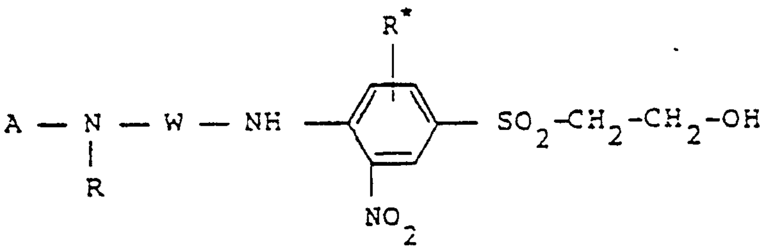

(2)

(dans laquelle R, R* et W ont les sens indiqués à la revendication 1, et A représente un atome d'hydrogène ou un reste acyle et, au cas où W représente un reste carbocyclique aromatique, A représente obligatoirement un reste acyle), on réduit le groupe nitro pour obtenir un groupe amino et l'on diazote le composé de formule générale (3) :

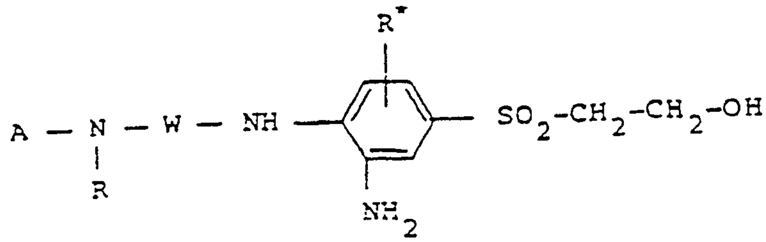

(3)

(dans laquelle A, R, R* et W ont les sens indiqués à la revendication 1) et éventuellement, au cas où le groupe A représentait dans la formule (3) un reste acyle, on enlève le groupe acyle du composé acylbenzotriazole pour obtenir un composé ou dérivé de benzotriazole, de formule générale (1).

7. Utilisation d'un composé selon l'une au moins des revendications 1 à 6 pour la synthèse de colorants.

8. Utilisation selon la revendication 7, pour la synthèse de colorants de la série de la triphénodioxazine pouvant réagir avec les fibres.

**Revendications pour l'Etat contractant suivant : ES**

1. Procédé pour préparer un composé répondant à la formule générale (1) :

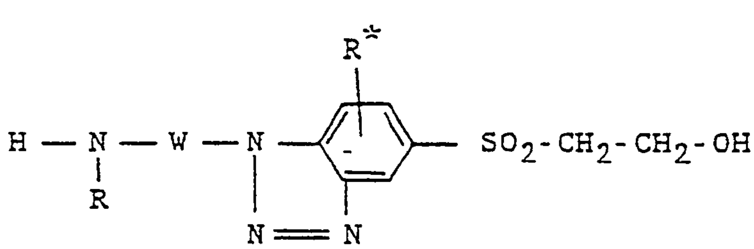

(1)

:

(dans laquelle :

R représente un atome d'hydrogène ou un groupe alkyle ayant 1 à 6 atomes de carbone, qui peut être substitué par un ou deux substituants choisis parmi un groupe hydroxy, sulfo, carboxy, phosphono et cyano,

W représente le reste éthylène-1,2, propylène-1,3, butylène-1,4, pentylène-1,5, hexylène-1,6, propylène-1,2, butylène-1,2, butylène-1,3, méthyl-2 propylène-1,3, hydroxy-2 propylène-1,3, carboxy-2 pentylène-1,5 phényl-2 propylène-1,3 ou W représente un reste phényl-2 propylène-1,3 à substituants sulfo sur le reste benzénique, ou bien W représente un reste répondant à l'une des formules générales (1a), (1b), (1c) ou (1d)

$$-(CH_2)_2-X-(CH_2)_2- \qquad (1a)$$

$$-(CH_2)_3-X-(CH_2)_3- \qquad (1b)$$

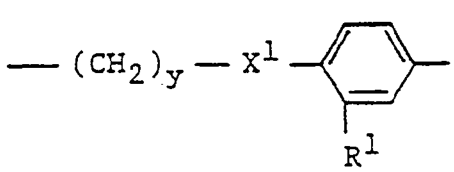

(1c)

$$-(CH_2-CH_2-NH)_n-CH_2-CH_2- \qquad (1d)$$

dans lesquelles :

X représente un atome d'oxygène, un atome de soufre, un groupe sulfonyle ou un groupe de formule -NH-, -N($CH_3$)- ou -N($COCH_3$)-,

$R^1$ représente un atome d'hydrogène ou un groupe sulfo,

n est un nombre valant 2, 3 ou 4,

$X^1$ représente le groupe -NH- ou un atome d'oxygène-, et

y est un nombre valant 2 ou 3,

ou bien W représente le reste cyclohexylène,-1,3 cyclohexylène-1,4, bis-(cyclohexylène-1,4)-méthane, diméthylène-1,8 naphtalène, di-méthylène-1,4 benzène, di-méthylène-1,3 benzène, bis-(éthylène-1',2')-1,4 pipéridine, phénylène-1,4 ou phénylène-1,3 ou un reste de formule générale (1e) ou (1f) :

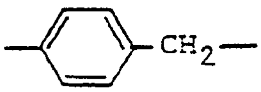
(1e)

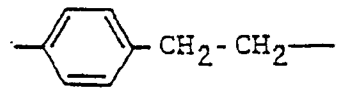
(1f)

et

R* représente un atome d'hydrogène ou un groupe alkyle ayant 1 à 6 atomes de carbone, un groupe alcoxy ayant 1 à 5 atomes de carbone, un atome d'halogène, un groupe carboxyle ou un groupe sulfo ), caractérisé en ce que, dans un composé de formule générale (2) :

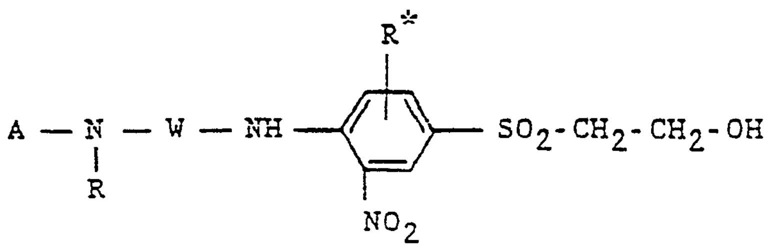
(2)

(dans laquelle R, R* et W ont les sens précités et A représente un atome d'hydrogène ou un reste acyle et, au cas où W représente un reste carbocyclique aromatique, A représente obligatoirement un reste acyle), on réduit le groupe nitro pour obtenir un groupe amino, et l'on soumet le composé de formule générale (3) :

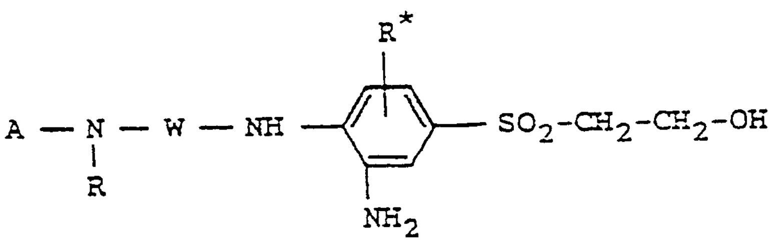
(3,

(dans laquelle A, R, R* et W ont les sens précités) à diazotation et éventuellement, au cas où, dans la formule (3), le groupe A représentait un reste acyle, on enlève ce groupe acyle du dérivé d'acylbenzotriazole pour obtenir un dérivé de benzotriazole répondant à la formule générale (1).

2. Procédé selon la revendication 1, caractérisé en ce que R* représente un atome d'hydrogène.

3. Procédé selon la revendication 1 ou 2, caractérisé en ce que R représente un atome d'hydrogène.

4. Procédé selon l'une au moins des revendications 1 à 3, caractérisé en ce que W représente un reste para- ou métha-phénylène.

5. Procédé selon l'une au moins des revendications 1 à 3, caractérisé en ce que W représente un reste éthylène-1,2, propylène-1,3, butylène-1,4 ou un reste isopropylène.

6. Utilisation d'un composé obtenu selon l'une au moins des revendications 1 à 5, pour la synthèse de colorants.

7. Utilisation selon la revendication 6, pour la synthèse de colorants de la série de la triphénodioxazine pouvant réagir avec les fibres.